# EUROPEAN PATENT SPECIFICATION

(11) **EP 2 844 164 B1**
(45) Date of publication and mention of the grant of the patent: **29.06.2016**
(21) Application number: 13724954.6
(22) Date of filing: 03.05.2013
(51) Int. Cl.: A61B 17/29, A61B 17/00

(54) **SURGICAL TOOL**
CHIRURGISCHES INSTRUMENT
OUTIL CHIRURGICAL

(30) Priority: 04.05.2012 US 201261642782 P
(43) Date of publication of application: 11.03.2015
(73) Proprietor: Agile Endosurgery, Inc., Chapel Hill, North Carolina 27514 (US)
(72) Inventor: STEEGE, Adam T. C., Chapel Hill, North Carolina 27514 (US)
(74) Representative: Awapatent AB
(86) International application number: PCT/US2013/039501
(87) International publication number: WO 2013/166409

(56) References cited:
- WO-A1-2011/109640
- WO-A2-2013/009699
- US-A1- 2005 096 694
- US-A1- 2010 160 929
- US-A1- 2010 262 180
- US-A1- 2011 022 078

## Description

### Background

Embodiments described herein generally relate to surgical apparatus for tissue and suture manipulation, and more particularly to apparatus that may be applied to conducting laparoscopic and endoscopic surgery.

Minimally invasive (endoscopic) surgery encompasses a set of techniques and tools, which are becoming more and more commonplace in the modem operating room. Minimally invasive surgery causes less trauma to the patient when compared to the equivalent invasive procedure. Hospitalization time, scarring, and pain are also decreased, while recovery rate is increased.

Endoscopic surgery is accomplished by the insertion of a trocar containing a cannula to allow passage of endoscopic tools. Optics for imaging the interior of the patient, as well as fiber optics for illumination and an array of grasping and cutting devices are inserted through a multiple cannulae, each with its own port.

Currently the majority of cutting and grasping tools are essentially the same in their basic structure. Standard devices consist of a user interface at the proximal end and an end effector at the distal end of the tool used to manipulate tissue and sutures. Connecting these two ends is a tube section, containing cables or rods used for transmitting motion from the user interface at the proximal end of the tool to the end effector at the distal end of the tool. The standard minimally invasive devices (MIDs) provide limited freedom of movement to the surgeon. The cannula has some flexibility of movement at the tissue wall, and the tool can rotate within the cannula, but such tools cannot articulate within the patient's body, limiting their ability to reach around or behind organs or other large objects. Several manually operated devices have attempted to solve this problem with articulated surgical tools that are controlled much in the same way as standard MIDs. These devices have convoluted interfaces, making them more difficult to control than their robotic counterparts. Many lack torsional rigidity, limiting their ability to manipulate sutures and denser tissue.

Robotic surgical instruments have attempted to solve the problems that arise from the limitations of standard MIDs with telemetrically controlled articulated surgical tools. However, these tools are often prohibitively expensive to purchase and operate. The complexity of the devices raises the cost of purchasing as well as the cost of a service contract. These robotic solutions may also have several other disadvantages such as complications during the suturing process and in some cases a lack of haptic feedback.

In the case of both articulated hand-held devices and robotic devices, the issue of compactness and strength are high priorities in terms of design. Many previously proposed articulated devices require a significant amount of space to articulate properly.
US2005096994 A1 discloses an endoscopic or laparoscopic instrument includes a distal tool, a rigid or flexible elongated shaft that supports the distal tool, and a proximal handle or control member, where the tool and the handle are coupled to the respective distal and proximal ends of the elongated shaft via bendable motion members. The tool and the tool motion member are coupled to the handle and the handle motion member via cables and a push rod in such a way that the movement of the handle with respect to the elongated shaft in any direction is replicated by the tool at the distal end of the shaft.
US 2010262180 A1 discloses an articulating mechanism useful, for example, for remote manipulation of various surgical instruments and diagnostic tools within, or to, regions of the body. Movement of segments at the proximal end of the mechanism results in a corresponding, relative movement of segments at the distal end of the mechanism. The proximal and distal segments are connected by a set of cables in such a fashion that each proximal segment forms a discrete pair with a distal segment.
US2011022078 A1, on which the preamble of claim 1 is based, discloses an articulating mechanism for use in remote guidance and manipulation of surgical or diagnostic tools. The mechanism having at least one pair of spherical joints interconnected by a set of tension members. Each joint includes a ball member, a socket member configured to pivotably receive at least a portion of the ball member, and at least one tension member extending through both the ball and socket members parallel to and offset from a central longitudinal axis of the joint.

### Summary

The surgical instrument according to the invention is disclosed in claim 1. Preferred embodiments are disclosed in the dependent claims.

Surgical instruments are disclosed for use in a wide variety of roles including grasping, dissecting, clamping, or retracting materials or tissue during surgical procedures performed within a patient's body and particularly within the abdominal cavity.

The distal joint in one embodiment is controlled by four cables, which in turn also control the jaws. There are three primary motions that these cables actuate: rotation about a primary joint axis, rotation about a secondary joint axis, and the opening and closing of the jaws. The embodiment described below is such that the end effector may be controlled by a manual interface or a robotic interface.

The instrument described below is one embodiment that can control the joint and jaws manually. The four cables that control the distal joint and jaws pass through the endoscopic tube section to the proximal joint. In one aspect, the cables terminate in the manipulator and the end effector. The cables follow guides in a path partially around each of a first central feature and a second central feature.

A second embodiment is also described in which four cables control the proximal and distal joints and a fifth cable passes through the center of these joints to control the jaws. The joint control cables terminate in the proximal and distal joints, while the jaw control cable passes through these joints and terminates in the jaw control pin at the distal end and the trigger at the proximal end. Additionally, in either of these embodiments the four joint control cables may be designed as a composite actuation system wherein the jaw assembly and distal joint contain cables that connect to rods in the tube portion which in turn connect to cables that continue into the proximal joint and interface assembly. The added rigidity of the rod portion of this actuation system may reduce backlash in the instrument as a whole.

The jaws may be of any of a variety of configurations. They may be tailored to a specific task, such as suture grasping, tissue grasping, tissue dissection or electrocautery. The embodiment described below is such that all of these specific tasks can be easily adapted to the current description.

Further, in one aspect an endoscopic surgical grasper is provided with a joint such that the grasper can articulate with two degrees of freedom.

In another aspect, the surgical grasper may be controlled robotically.

In another aspect, the surgical grasper may be controlled by a manual interface.

In another aspect, an endoscopic surgical end effector is provided that is adaptable to multiple different jaw structures for different surgical procedures.

In another aspect, an endoscopic surgical instrument is provided that utilizes a proximal joint and interface to control a distal joint and jaws for performing a variety of surgical tasks.

In another aspect, a hybrid actuation system is provided that utilizes a combination of rods and cables to provide articulation control with reduced backlash as compared with cables alone.

Further features of the subject invention will become more readily apparent from the following detailed description taken in conjunction with the accompanying drawings.

### Brief Description Of The Drawings

Preferred embodiments of the subject invention will be described hereinbelow with reference to the drawings, wherein:
FIG. 1 is a right side perspective view of an embodiment of a surgical instrument.
FIG. 2 is a right side exploded perspective view of the surgical instrument as shown in FIG. 1.
FIG. 3 is a right side perspective view of the surgical instrument as shown in FIG. 1 in an articulated position.
FIG. 4 is a right side elevation view of the surgical instrument as shown in FIG. 1 in an articulated position about its primary axes.
FIG. 5 is a top plan view of the surgical instrument as shown in FIG. 1 in an articulated position about its secondary axes.
FIG. 6 is a right side perspective view of a distal end of the surgical instrument as shown in FIG. 1.
FIG. 7 is a right perspective view of a distal end of the surgical instrument as shown in FIG. 6 showing the cabling system.
FIG. 8 is a right side exploded perspective view of a distal end of the surgical instrument as shown in FIG. 7.
FIG. 9 is a right side perspective view of a distal joint for use in the surgical instrument as shown in FIG. 1.
FIG. 10 is a right side exploded perspective view of the distal joint as shown in FIG. 9.
FIG. 11 is a top exploded perspective cross-section view of the joint as shown in FIG. 10.
FIG. 12 is a right side exploded perspective cross-section view of the joint as shown in FIG. 10.
FIGs. 13-16 are views of a ball for use in a joint as shown in FIG. 9.
FIG. 17 is a right side perspective exploded perspective view of the joints of the cabling system as shown in FIG. 7.
FIG. 18 is right side elevation view of a proximal end of the surgical instrument as shown in FIG. 1.
FIG. 19 is a right side exploded elevation view of an interface assembly of the proximal end of the surgical instrument as shown in FIG. 1.
FIG. 20 is a right side perspective view of the cabling system as shown in FIG. 7 in an articulated position about its primary axes.
FIG. 21 is a right side perspective view of the cabling system as shown in FIG. 7 in an articulated position about its secondary axes.
FIG. 22 is a right side perspective view of the cabling system as shown in FIG. 7 with the jaws in an open position.
FIG. 23 is a right side section view of the interface assembly of the surgical instrument as shown in FIG. 1 with the trigger in an open position.
FIG. 24 is a right side section view of the interface assembly of the surgical instrument as shown in FIG. 1 with the trigger in a closed position.
FIG. 25 is a right side perspective view of an alternate embodiment of a distal joint for use in the surgical instrument as shown in FIG. 1.
FIG. 26 is a right side exploded perspective view of the joint as shown in FIG. 25.
FIG. 27 is an exploded top section view of the joint as shown in FIG. 25.
FIG. 28 is an exploded right section view of the joint in FIG. 25.
FIG. 29 is a right perspective view of an alternate embodiment of a cabling system and jaw assembly for use in a surgical instrument as shown in FIG. 1.
FIG. 30 is an exploded perspective view of the cabling system and the jaw assembly as shown in FIG. 29.
FIG. 31 is a right side perspective view of the cabling system and jaw assembly as shown in FIG. 31 with the jaws in an open position.
FIG. 32 is a right side section view of an alternate embodiment of the interface assembly for use in the surgical instrument as shown in FIG. 1 that corresponds to the embodiment of the cabling system and jaw assembly as shown in FIG. 29.
FIG. 33 is a right side perspective view of the distal portion an alternate embodiment of the surgical instrument as shown in FIG. 1 with the tube removed.
FIG. 34 is a right side perspective view of the proximal portion of the embodiment of the surgical instrument as shown in FIG. 33.

### Description

Embodiments of a surgical instrument are disclosed for use in a wide variety of roles including, for example, grasping, dissecting, clamping, electrocauterizing, or retracting materials or tissue during surgical procedures performed within a patient's body.

Certain terminology is used herein for convenience only and is not to be taken as a limitation. For example, words such as "upper," "lower," "left," "right," "horizontal," "vertical," "upward," and "downward" merely describe relative positions or the configuration shown in the Figures. The components may be oriented in any direction and the terminology, therefore, should be understood as encompassing such variations unless specified otherwise.

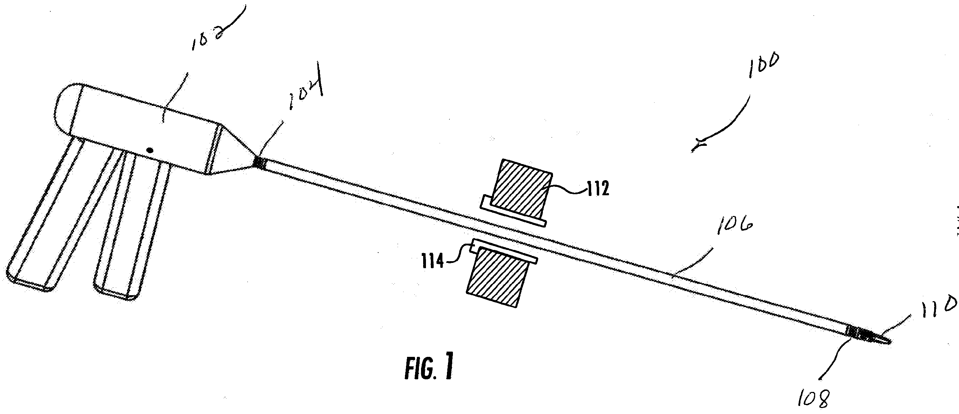

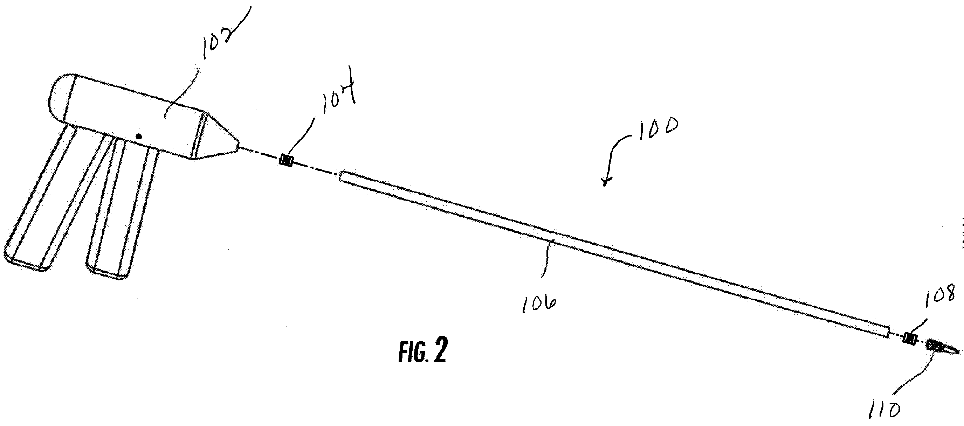

Referring now to the drawings, wherein like reference numerals designate corresponding or similar elements throughout the several views, an embodiment of a surgical instrument or tool is shown in FIGs. 1 and 2 and is generally designated at 100. The surgical instrument 100 comprises an operator interface, or manipulator, 102 designated to be at a proximal end of the tool 100, a proximal joint 104 mounted to the manipulator 102, an elongated, hollow member or tube 106 one end of which is mounted to the proximal joint 104, a distal joint 108 mounted to the other end of the tube 106, and an end effector 110 mounted to the distal joint 108 and designated to be at a distal end of the tool 100. The components of the embodiments of the surgical tool 100 described herein are largely symmetric about a vertical plane. A front of the device is designated as a front of the end effector 110 and other directions, which are intended as a means for comprehension of the design and not to constrain the design, are derived from this designation. The majority of views are given from a right perspective, as many of the components and assemblies are symmetrical. Features of asymmetric components are clarified with further views.

The components of the surgical instrument 100 may be formed from a rigid, durable material such as, for example, stainless steel, rigid plastic and the like. It is understood that the scope of the embodiments of the surgical instrument 100 is not intended to be limited by the materials listed here, but may be carried out using any material which allows the construction and operation of the surgical instrument 100 described herein.

The manipulator 102 and the end effector 110 are operatively connected with control cables contained within the tube 106, as described herein below. The control cables may be stainless steel rope, aramid fiber cables, aligned polymer fiber cables, or the like. The manipulator 102 is gripped by in a hand of a user, such as a surgeon. When the surgeon actuates the manipulator 102, the end effector 110 has corresponding movements. The surgical instrument 100 is shown in use in FIG. 1 with a portion of the tube 106, the distal joint 108, and the end effector 110 having passed through a tissue wall 112 via a cannula 114. The surgical instrument 100 is in a neutral position, not articulated, with the manipulator 102 and the end effector 110 in a closed position.

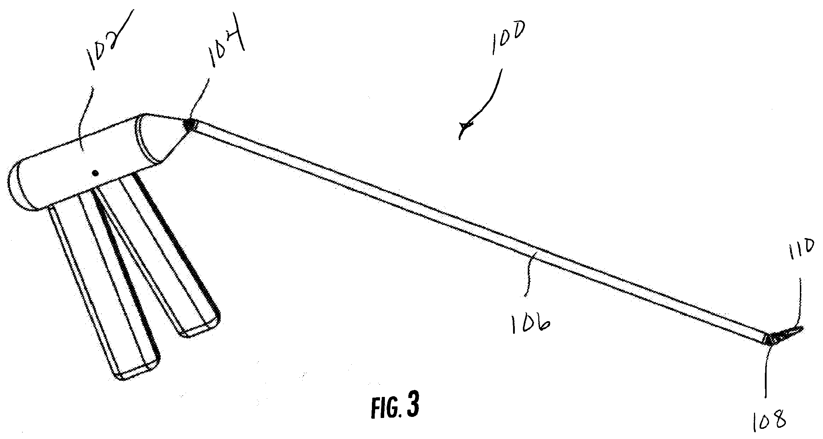

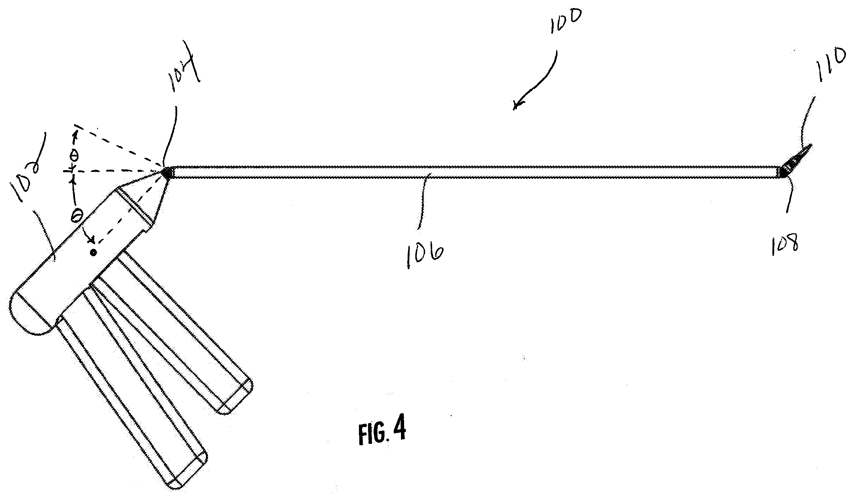

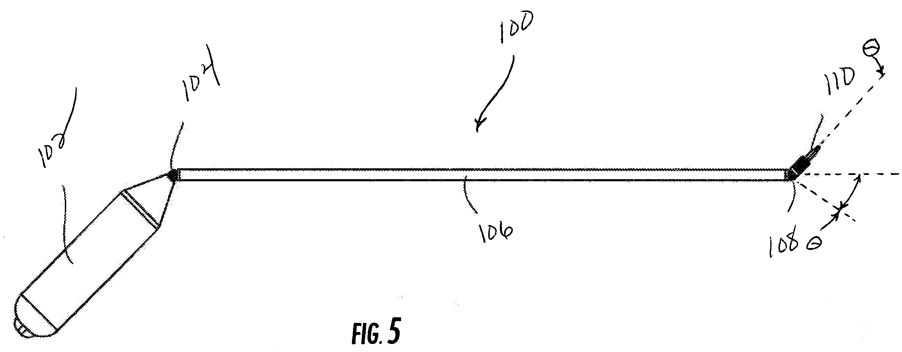

The cabling arrangement enables the surgeon to angle the manipulator 102 with his or her hand relative to the proximal joint 104 to cause the distal joint 108 to move in a similar manner in the opposite direction, imitating the surgeon's movements and providing directional control of the distal portion of the surgical instrument 100. Such corresponding pivoted positions of the manipulator 102 and the end effector 110 relative to the longitudinal axis of the tube 106 are shown in FIGs. 3-5, which show the manner in which the surgical instrument 100 articulates when the interface assembly 102 is moved by the user to an angle relative to the tube 106. The end effector 110 is moved in the opposite direction to the same angle relative to the tube 106. Thus, consistent alignment is maintained between the interface assembly 102 and the end effector 110. The maximum angle of deflection θ in every direction from the longitudinal axis of the tube 106, such as between top and bottom as shown in FIG. 4 and side-to-side in FIG. 5, shows the range of motion at each end of the surgical instrument 100. The range of motion is determined by the design of the proximal joint 104 and the distal joint 108 and the direction of deflection, and may vary from the approximately 45 degrees that is shown in the FIGs.

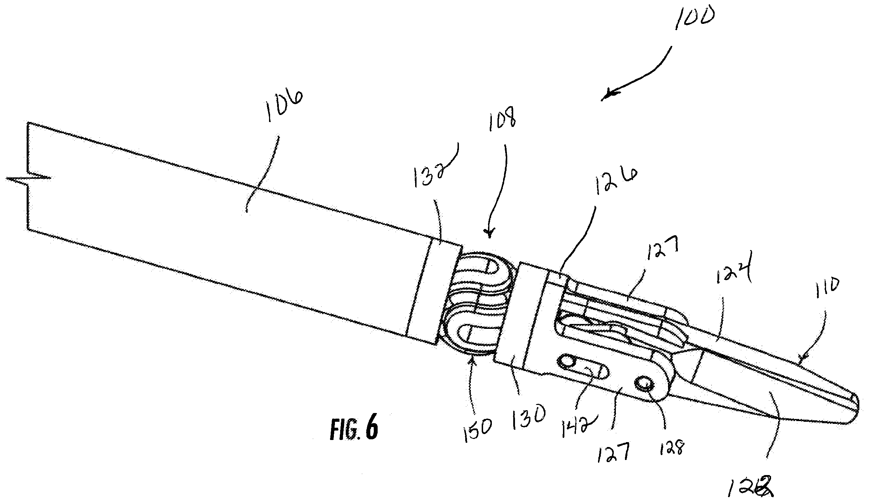

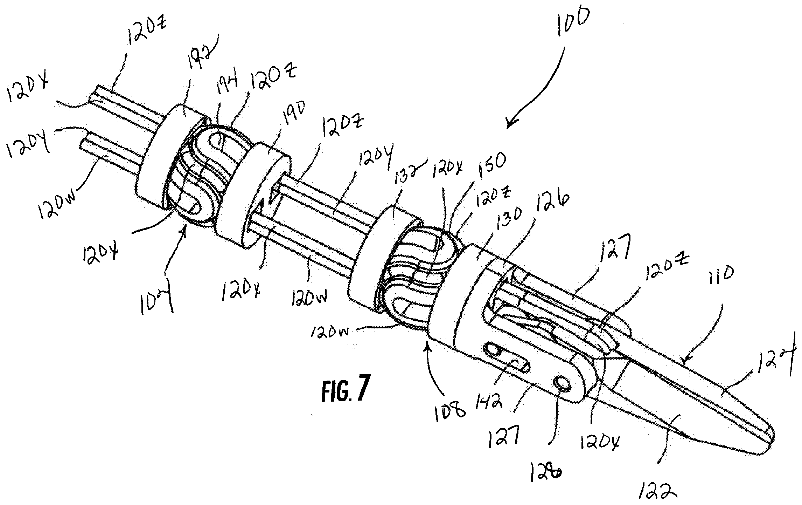

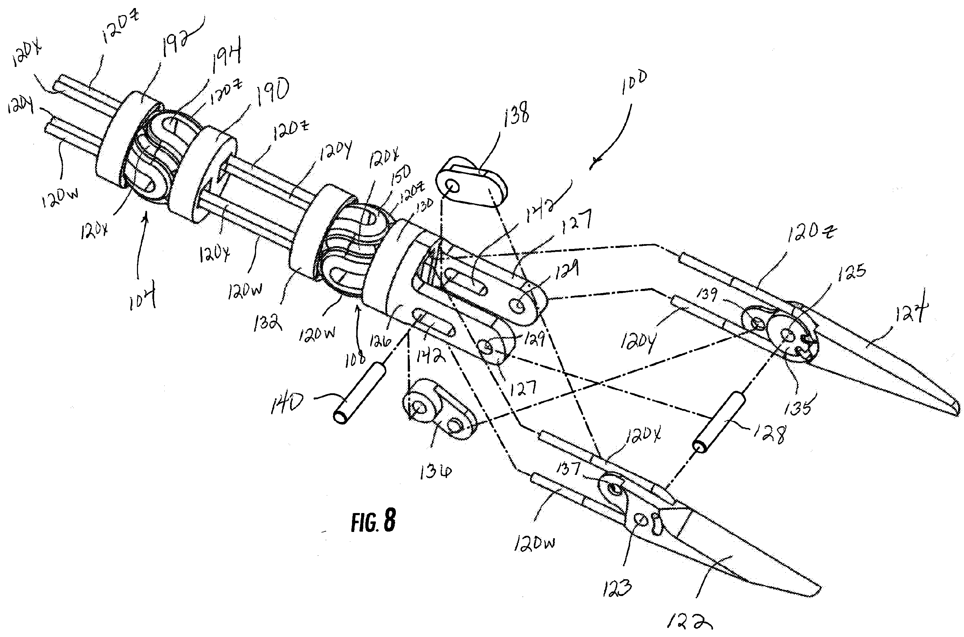

FIGs. 6-8 show the distal end of the surgical instrument 100. In FIGs. 7 and 8, the tube 106 is removed to expose the arrangement of the proximal joint 104, the distal joint 108, the end effector 110 and four control cables 120w, 120x, 120y, 120z. As shown in FIGs. 6-8, the end effector 110 comprises a first jaw element 122, a second jaw element 124, a jaw base 126, a first constraining link 136 and a second constraining link 138. The first and second jaw elements 122, 124 are pivotally connected to the jaw base 126 by a primary jaw pin 128 in holes 123, 125 in the proximal ends of the first and second jaw elements and holes 129 in the distal ends of opposed, distally extending ears 127 of the jaw base 126.

The four control cables 120w, 120x, 120y, 120z connect to protruding round features 134, 135 on the inside of the first and second jaw elements 122, 124. Only the round feature 135 associated with the second jaw element 124 is visible in FIG. 8. Since the end effector 110 is rotationally symmetric about its longitudinal axis, the first jaw element 122 has the same round feature 134 as the second jaw element 124 but has been rotated 180 degrees about its longitudinal axis.

Two cables 120w, 120x engage the round feature of the first jaw element 122. The other two cables 120y, 120z engage the round feature 135 of the second jaw element 124. It is understood that the cable 120y follows a path that mirrors the path of cable 120w. When cables 120w and 120z are retracted, the first and second jaw elements 122,124 are moved to an open position. When cables 120x and 120y are retracted, the first and second jaw elements 122, 124 are moved to a closed position.

Opposed constraining links 136,138 each has a distal transverse post received in corresponding openings 137, 139 at the proximal ends of the first and second jaw elements 122, 124. A sliding pin 140 connects the constraining links 136, 138 via slots 142 in the ears 127 of the jaw base 126. The constraining links 136, 138 can thus translate in a longitudinal direction within the slots relative to the jaw base 126. Specifically, when the first jaw element 122 rotates in a clockwise direction, the first constraining link 136 rotates such that the sliding pin 140 translates longitudinally in the slots 142 in a distal direction. This will in turn similarly move the second constraining link 138 causing the second jaw element 124 to rotate in a counterclockwise direction. Thus, the described configuration of the linkage system constrains the first and second jaw elements 122,124 such that they may only move in opposite directions.

The jaw base 126 is mounted to a distal end 130 of the distal joint 108. The proximal end 132 of the distal joint 108 is in turn mounted to the distal end of the tube 106. The proximal joint 104 and the distal joint 108 are operatively connected with the cables 120w, 120x, 120y, 120z such that movement of the proximal joint 104 controls the movement of the distal joint 108. The proximal joint 104 and the distal joint 108 may be joints that allow pivoting about two intersecting, perpendicular axes, and provide two degrees of freedom, being free to move in any combination of directions deflecting relative to the longitudinal axis of the tube 106, such as, for example, a ball-and-socket joint.

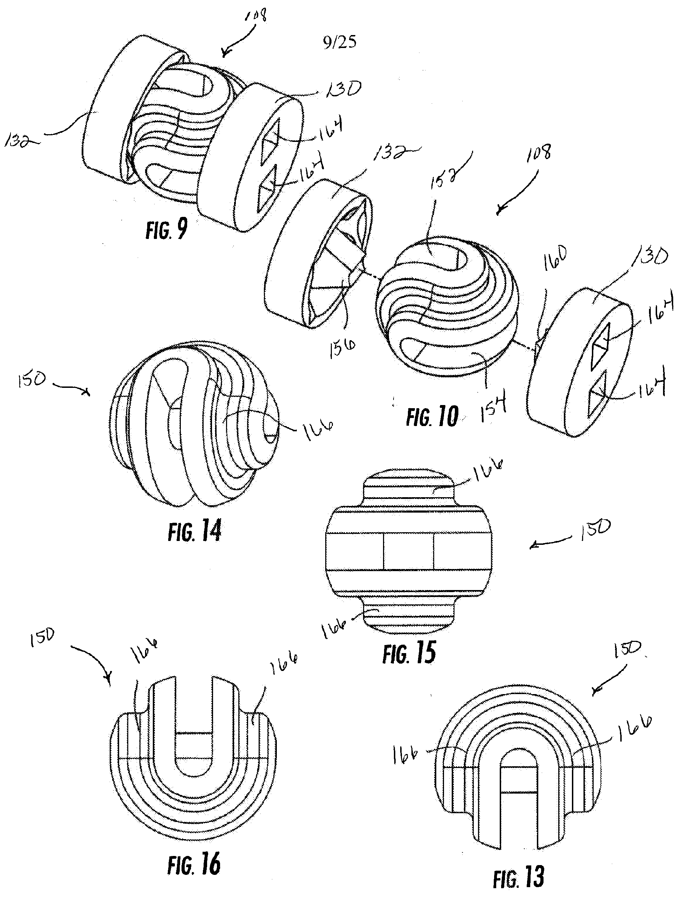

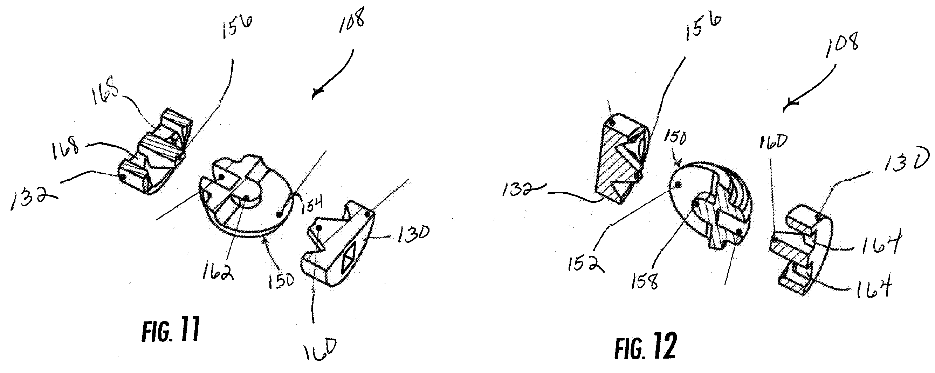

FIGs. 9-12 show the structure of the distal joint 108. The components of the proximal joint 104 are identical to the components of the distal joint 108. However, a proximal joint ball 194 and a distal joint ball 150 face opposite directions in use, such that they mirror each other. All components of both the proximal and distal joints 104, 108 are symmetric about perpendicular longitudinal planes. As seen in FIGs. 9-12, the distal joint 108 is a ball-and-socket joint comprising a distal end base 130, a ball 150 and a proximal end base 132. The ball 150 (FIGs. 13-16) has a proximal slot 152 and an opposed distal slot 154 which is perpendicular to the proximal slot 152. The proximal slot 152 receives a central axial tab 156 extending distally from the proximal end base 132 for engaging a round feature 158 in the slot 152. The end of the tab 156 has a corresponding curved surface for smooth movement against the round feature 158 in the ball 150. In this arrangement, the ball 150 can pivot relative to the proximal end base 132 about a primary axis perpendicular to the longitudinal axis of the round feature 158. The distal end base 130 has a similar central axial tab 160 extending proximally from the distal end base 130. The end of the tab 160 has a curved surface that engages and moves against a second round feature 162 in the slot 154. The distal end base 130 may thus pivot relative to the ball 150 about a secondary axis perpendicular to the second round feature 162 in the ball 150. This configuration of cooperating tabs and slots for modifying a standard ball-and-socket joint allows for precise linear control of the relative position of the components, as well as improved transmission of torsional motion as compared with a standard ball-and-socket joint. It is understood that the ball 150 need not be a ball shape, but may be any configuration that provides articulation of the components about two perpendicular axes, which may be intersecting axes as configured, and may include round features.

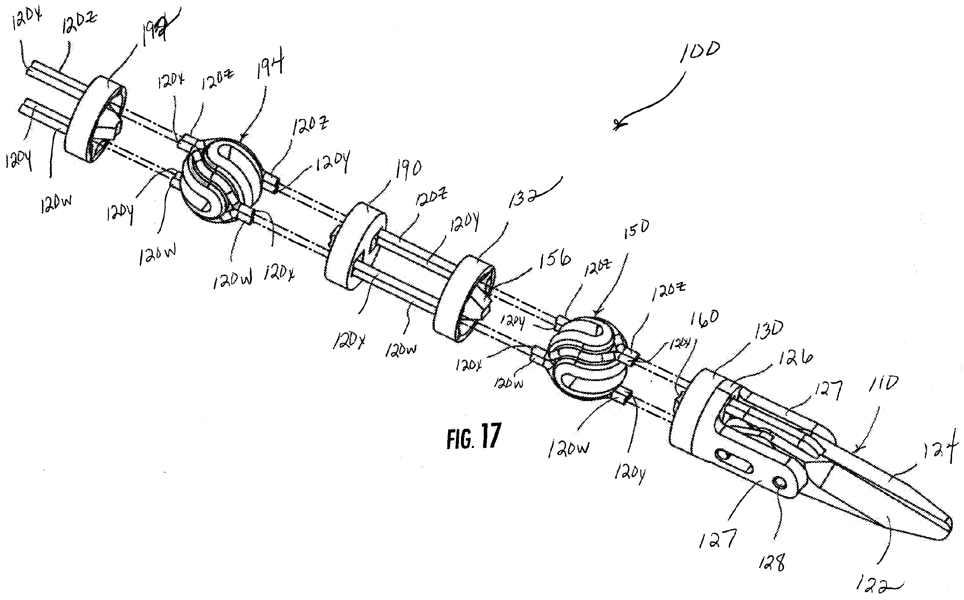

Referring to FIG. 17, the control cables 120w, 120x, 120y, 120z extend proximally from the end effector 110 and through two openings 164 in the distal end base 130 of the distal joint 108. The openings 164 are radially spaced from opposite sides of the central axial tab 160. The ball 150 defines a continuous oval groove 166 in the surface of the ball 150. The groove 166 functions as a cable guide. The ball 150 is symmetric about both its central perpendicular planes such that the groove 166 presents identical features functioning as cable guides on the opposite faces of the ball 150.

The control cable 120w exits a slot in the jaw base 126 and enters an opening 164 in the distal end base 130 of the distal joint 108. The control cable 120w passes around the groove 166 on the bottom surface and side of the ball 150 and passes through an opening 168 in the proximal end base 132 of the distal joint 108. After exiting the distal joint 108, the control cable 120w continues through an opening 170 in the distal end base 190 of the proximal joint 104, along a groove 174 in a ball 194 and through an opening 172 in the proximal end base 192 of the proximal joint 104.

The control cable 120x exits the same slot in the jaw base 126 as control cable 120w and enters the opposite opening 164 in the distal end base 130 of the distal joint 108. The control cable 120x passes around the groove 166 on top of the ball 150 and passes through the same opening 168 in the proximal end base 132 of the distal joint 108 as control cable 120w. After exiting the distal joint 108, the control cable 120x continues through the same opening in the distal end base 190 of the proximal joint 104. The control cable 120x passes over the groove 174 in the proximal ball 194 and through an opening 172 in the proximal end base 192 of the proximal joint 104 opposite from the opening for the control cable 120w.

Control cable 120y exits a slot in the jaw base 126 opposite the slot passing the control cables 120w, 120x. The control cable 120y passes through the same opening 164 in the distal end base 130 as the control cable 120w. The control cable 120y passes around the groove 166 on the bottom surface and side of the ball 150 and passes through an opening 168 in the proximal end base 132 opposite the opening passing the control cables 120w, 120x. After exiting the distal joint 108, the control cable 120y continues through an opening 170 in the distal end base 190 of the proximal joint 104. The opening 170 in the proximal end base 192 is opposite the opening passing the control cables 120w, 120x. The control cable 120y passes over the groove 174 in the proximal ball 194 and through an opening 172 in the proximal end base 192 of the proximal joint 104 which is the same opening for passing the control cable 120w.

Control cable 120z exits the same slot in the jaw base 126 as control cable 120y and passes through the same opening 164 in the distal end base 130 as control cable 120x. The control cable 120z passes in the groove 166 around the ball 150 and through the same opening in the proximal end base 132 of the distal joint 108 as the control cable 120y. After exiting the distal joint 108, the control cable 120z continues through the same opening 170 in the distal end base 190 of the proximal joint 104 as the control cable 120y. The control cable 120z passes over the ball 194 in the groove 174 and through the same opening 172 in the proximal end base 192 of the proximal joint 104 as the control cable 120x.

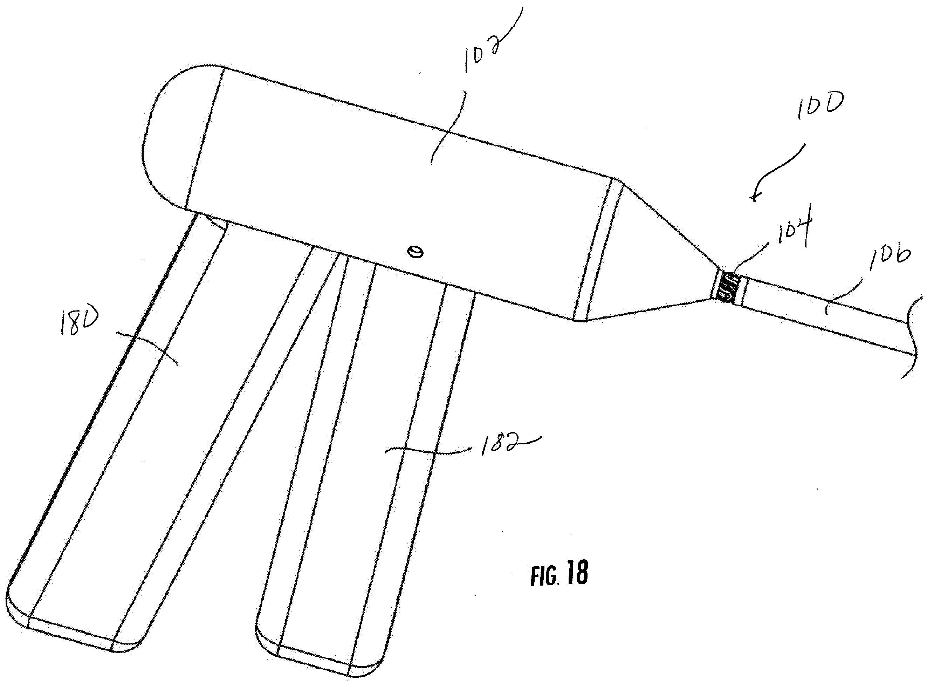

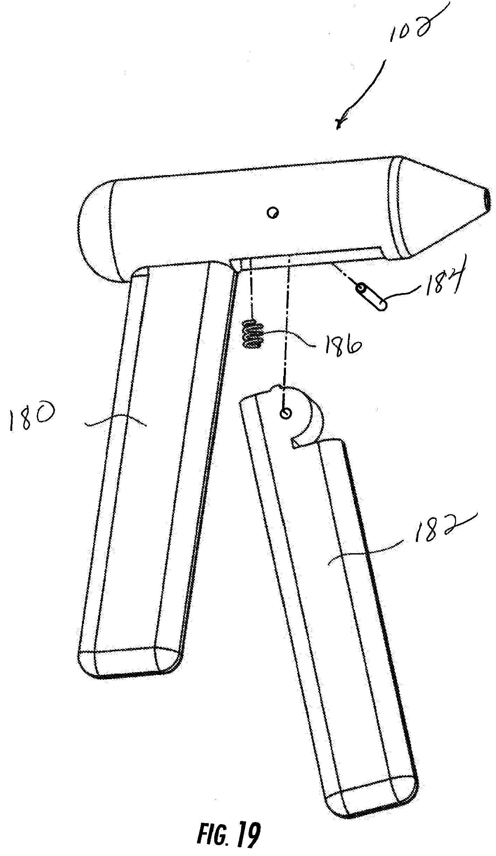

FIGs. 18 and 19 show the structure of the proximal end of the surgical instrument 100, including the interface or manipulator assembly 102. The manipulator assembly 102 comprises a handle 180 and a trigger 182 which is pivotally mounted to the handle 180 by a pin 184 that allows the trigger 182 to rotate about the pin 184. The trigger 182 is biased into a first home position by a spring 186.

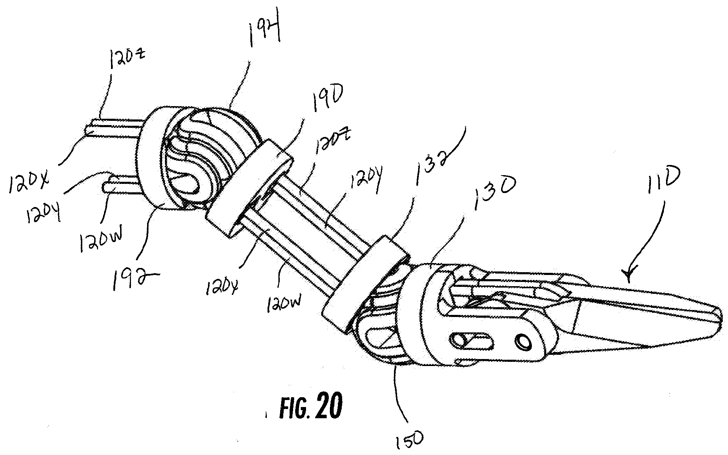

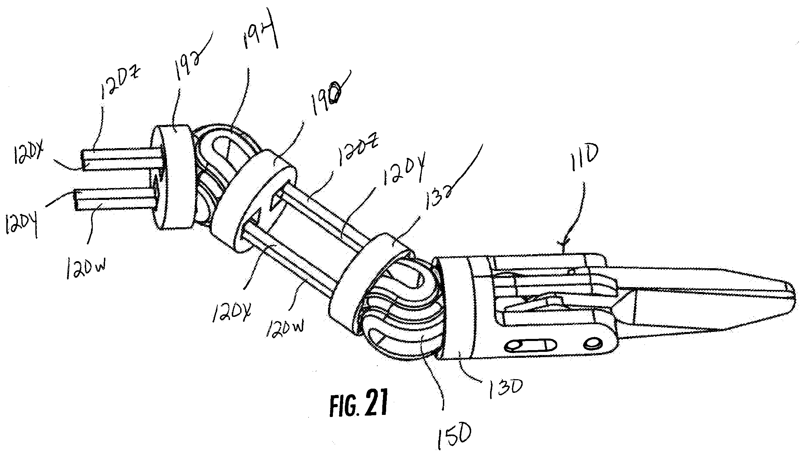

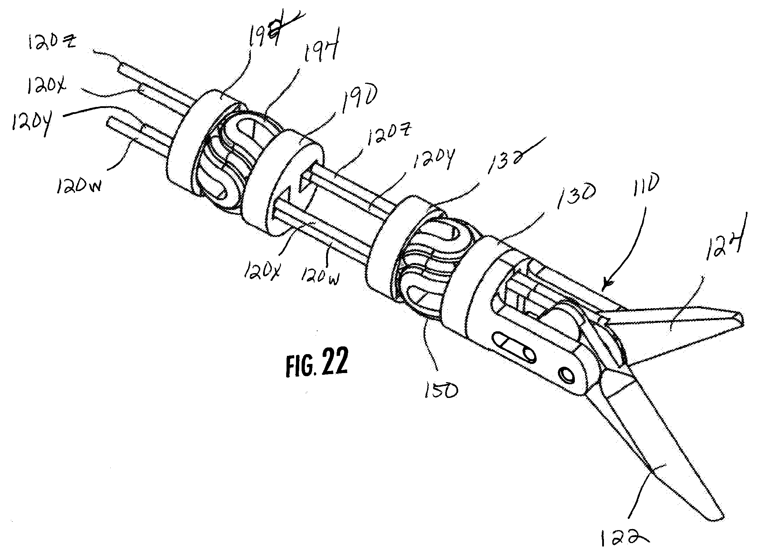

FIGs. 20-22 depict the manner in which the motion of the proximal joint 104 and the distal joint 108 and the control cables 120w, 120x, 120y, 120z combine to control articulation of the end effector 110. If control cables 120x and 120z are fixed relative to the proximal end base 192 of the proximal joint 104, then rotating the proximal end base 192 and the ball 194 of the proximal joint 104 about its primary axis will retract control cables 120z and 120z in the tube 106 portion of the surgical instrument 100. This will cause a corresponding rotation of the distal ball 150 and the proximal end base 132 of the distal joint 108. The end effector 110 will be unaffected by retraction of these cables due to the constraints applied by the linkage system.

If control cables 120y and 120z are fixed relative to the proximal end base 192 of the proximal joint 104, then rotating the proximal end base 192 about the secondary axis of rotation will retract control cables 102y and 120z in the tube 106 section of the surgical instrument 100, which will cause a corresponding rotation of the distal end base 130 of the distal joint 104. If cables 120w and 120z are retracted, this will not affect either the proximal joint 104 or the distal joint 108 since the control cables 120w and 120z are diagonally opposed and would act in opposition to one another to control either axis of motion of the proximal and distal joints 104, 108. This retraction produces an opening motion in the jaw elements 122,124 of the end effector assembly 110.

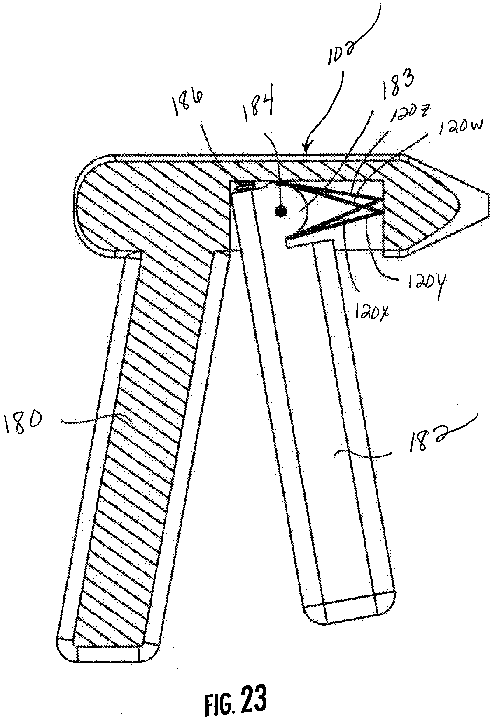

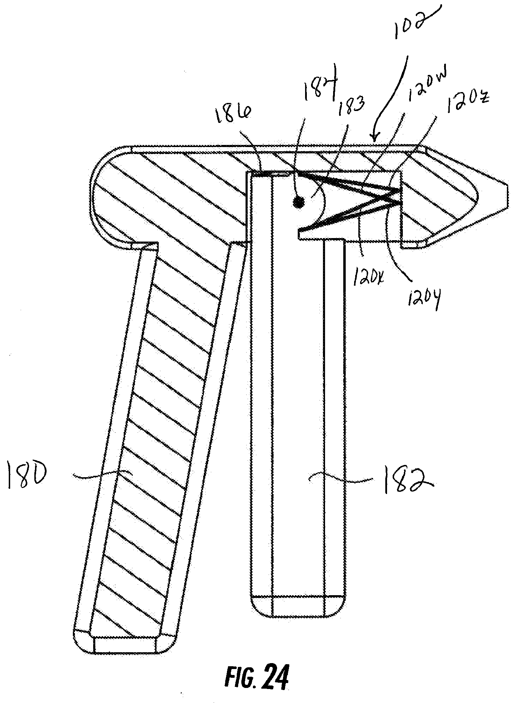

FIGs. 23 and 24 show two positions of the interface assembly 102 for producing the described cable retractions. After the control cables 120w, 120x, 120y, 120z exit the proximal end base 192, the control cables 120w, 120x, 120y, 120z enter the handle 180 and are connected to the trigger 182. Control cables 120w and 120z are connected at the top of a round feature 183 of the trigger 182. Control cables 120x and 120y are connected at the bottom of the round feature 183. If the trigger 182 is rotated clockwise to a closed position (FIG. 24), control cables 120x and 120y are retracted, which closes the jaw elements 122, 124. If the trigger 182 is rotated counterclockwise to an open position (FIG. 23), control cables 120w and 120z are retracted, which opens the jaw elements 122, 124. If the interface 102 and proximal end base 192 are rotated about the primary axis of the proximal joint 104, control cables 120x and 120z are retracted, as previously described. This will have no effect on the interface 102 as control cables 120x and 120z are in opposition to one another relative to the motion of the trigger 182. Similarly, motion about the secondary joint axes will not affect the interface 120 because control cables 120y and 120z are in opposition to each other relative to the motion of the trigger 182.

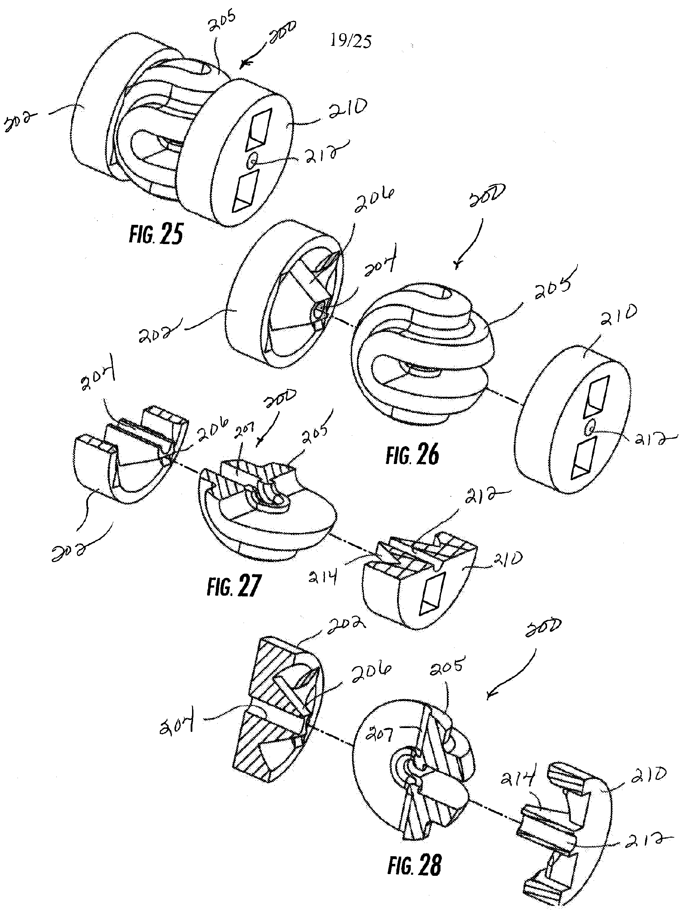

FIGs. 25-28 show an alternate embodiment of a ball-and-socket joint, generally designated at 200, for use in the surgical instrument 100. In this embodiment, a proximal end base 202 has a central axial passage 204 through a central, distally extending tab 206 to allow passage of a fifth central control cable 208. Similarly, the ball 205 has a pass through opening 207 and the distal end base 210 defines a central axial passage 212 through a central proximally extending tab 214. The series of openings through the components allow the central control cable 208 to pass completely through the joint 200.

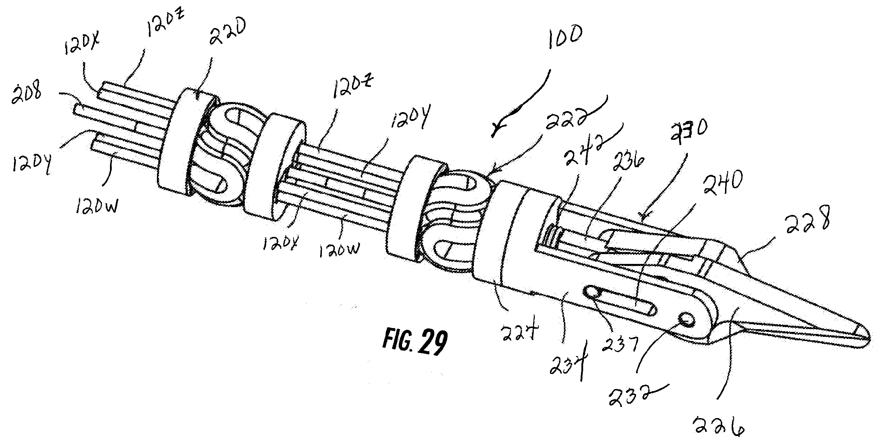

Referring to FIGs. 29-31, the four control cables 120w, 120x, 120y, 120z are used to control the proximal joint 220 and the distal joint 222 in a manner identical to the previous embodiment described hereinabove. The ends of the control cables 120w, 120x, 120y, 120z terminate in the distal end base 224 of the distal joint 222 and the interface handle 182.

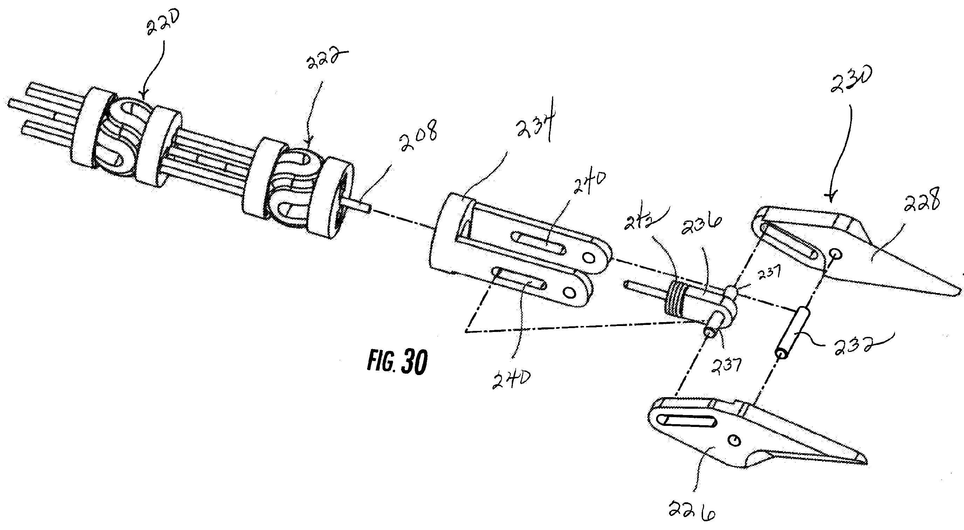

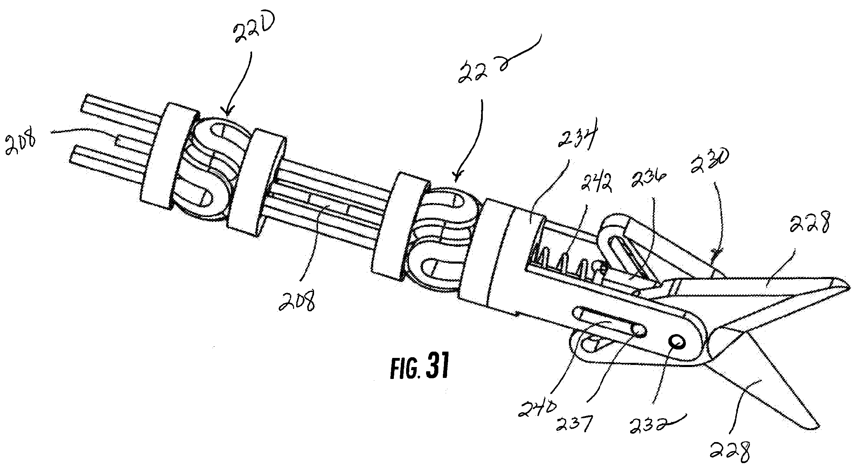

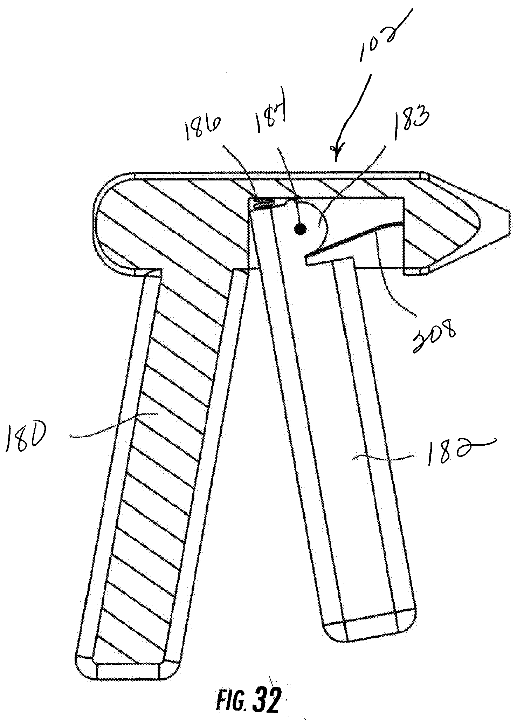

In the embodiment shown, a first jaw element 226 and a second jaw element 228 of an end effector assembly 230 are controlled by the central control cable 208 passing through the central axis of the joints 220, 222. Referring to FIG. 30, the jaw elements 226, 228 of the end effector assembly 230 are pivotally connected by a pin mounted in holes in ears of a jaw base 134. A jaw driver 136 comprises a transverse pin 137 extending through longitudinal slots in the proximal ends of the jaws 126, 128. The ends of the pin 137 are received in opposed slots 140 in the ears of the jaw base 234. The driver 236 is thus able to reciprocate relative to the jaw base 234 in a longitudinal direction. The driver 236 is distally biased by a spring 242 disposed between the jaw base 234 and the driver 236. The distal end of the central control cable 208 is connected to the driver 236. When the driver 136 translates longitudinally proximally, the jaws 226, 228 close (FIG. 29). When the driver 236 translates longitudinally distally the jaws 226, 228 open (FIG. 31). The translation of the driver 236 is controlled by the central control cable 208, which is in turn controlled by the trigger (FIG. 32). Thus, in this embodiment, only one cable 208 is needed to control the position of the jaw elements 226, 228.

In all embodiments described herein, the control cables may be affixed at their termination point by welding or other fusion method, by adhesive, or by swaging. For example, holes may be drilled in the trigger 182 to facilitate the swaging or adhesive attachment methods. Similar attachment methods may be used to attach the central control cable 208 to the driver 236. Other attachment methods may also be utilized depending on the material of the cables and other components of the surgical instrument 100.

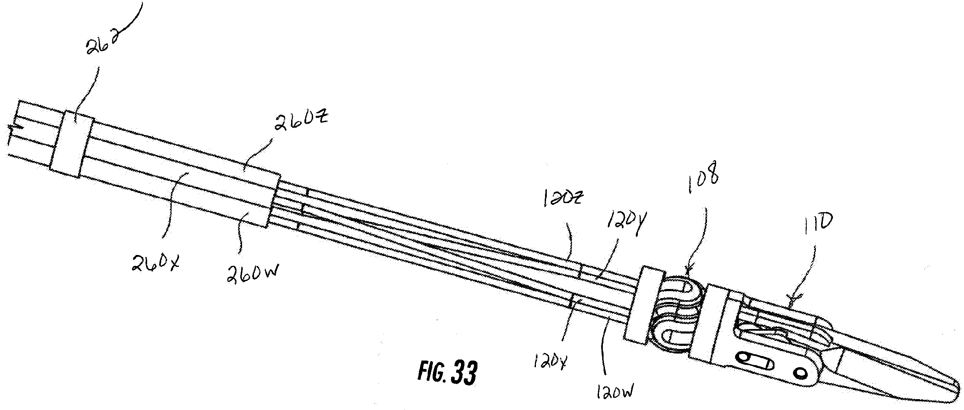

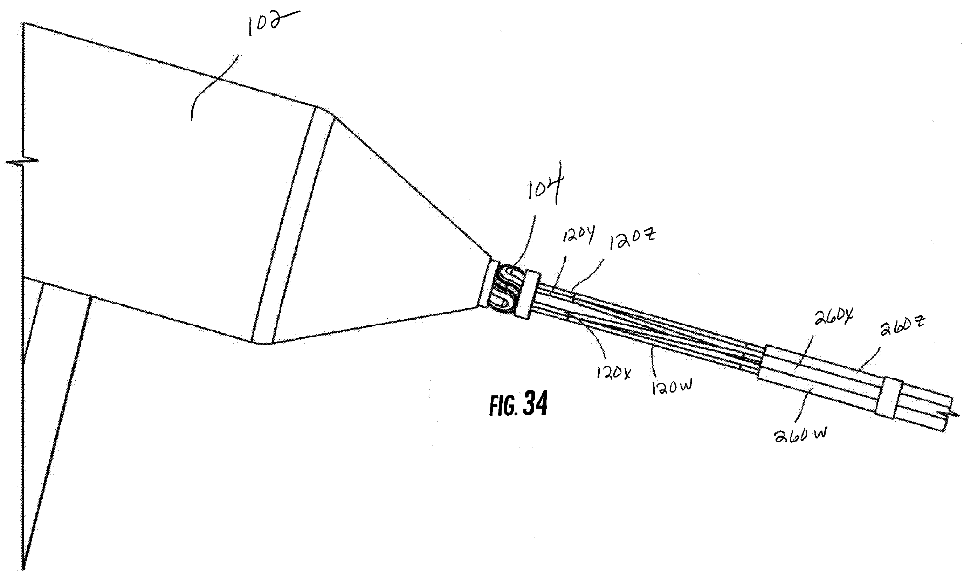

FIGs. 33 and 34 show a distal end 252 and a proximal end 254, respectively, of a hybrid actuation system 250. The hybrid actuation system uses both cables and solid rods to control the surgical instrument 100 described in the first embodiment. In the hybrid actuation system, ends of the four control cables 120w, 120x, 120y, 120z connect to four rods 260w, 260x, 260y, 260z positioned intermediate the lengths of the cables. Holes may be drilled in the ends of the rods 260w, 260x, 260y, 260z to facilitate the attachment of the control cables 120w, 120x, 120y, 120z by welding or other fusion methods, or by adhesive or swaging. It is understood that other suitable methods of attachment may also be utilized. The control rods 260w, 260x, 260y, 260z are slidingly received in a distal guide 262 and a spaced proximal guide 264, such that the rods 260w, 260x, 260y, 260z can reciprocate longitudinally relative to the guides 262, 264 in a proximal and or a distal direction. The operation and function of the surgical instrument 100 is unaffected by this configuration. However, backlash may be reduced by the using the rigid rods 260w, 260x, 260y, 260z to replace most of the lengths of control cables in the hybrid actuation system 250.

As described hereinabove, the distal joint 108 and the end effector 110 articulate in a direction opposite the direction of articulation of the interface assembly 102 and the proximal joint 104. This arrangement maintains a constant orientation of the end effector 110 relative to the interface assembly 102, providing simple control to the user. It is understood that the degree of articulation shown in the FIGs. is meant for demonstrative purposes and is not an indication of any limitation of the design. It is also understood that the design of the end effector in the first embodiment herein is meant to be generalized to any assembly utilizing four control cables for actuation which is constrained such that the first and second jaw elements 126, 128 may only move in opposite directions and may produce motion in a plurality of objects, which include but are not limited to cauterizing contacts, pliers, and scissor blades. It is further understood that the design of the end effector 230 in the second embodiment described herein is meant to be generalized to any assembly utilizing a single cable for actuation and for producing motion of a plurality of objects, which include but are not limited to cauterizing contacts, pliers, and scissor blades.

Although the surgical instrument 100 has been shown and described in considerable detail with respect to only a few exemplary embodiments thereof, it should be understood by those skilled in the art that I do not intend to limit the surgical instrument 100 to the embodiments since various modifications, omissions and additions may be made to the disclosed embodiments without materially departing from the novel teachings and advantages of the surgical instrument 100, particularly in light of the foregoing teachings.

## Claims

1. A surgical instrument (100) for use by an operator, comprising:
a manipulator (102) adapted to receive at least a portion of the operator's hand;
a proximal joint (104) having a first base and a second base and including a first central feature that provides for articulation about two perpendicular axes, the proximal joint (104) first base being mounted to the manipulator (102);
a hollow elongated member (106) having a first end, a second end, and a longitudinal axis, the elongated member (106) first end being mounted to the proximal joint (104) second base;
a distal joint (108) having a first base and a second base and including a second central feature that provides for articulation about two perpendicular axes, the distal joint (108) first base being mounted to the elongated member (106) second end;
an end effector (110) including at least one movable jaw (122, 124), the end effector (110) mounted to the distal joint (108) second base;
cables (120w, 120x, 120y, 120z) that engage the first central feature and the second central feature to operatively couple the manipulator (102), proximal joint (104), and distal joint (108);
the first central feature and second central feature defining guides in their surfaces to receive the cables (120w, 120x, 120y, 120z);
**characterized in that** the cables (120w, 120x, 120y, 120z) follow the guides in a path partially around each of the first central feature and the second central feature.

2. The surgical instrument (100) of claim 1, wherein the first central feature and the second central feature are substantially ball-shaped.

3. The surgical instrument (100) of claim 1, wherein the cables (120w, 120x, 120y, 120z) concurrently operatively couple the manipulator (102) and the end effector (110).

4. The surgical instrument (100) of claim 2, wherein the cables (120w, 120x, 120y, 120z) comprise four cables.

5. The surgical instrument (100) of claim 4, wherein the cables (120w, 120x, 120y, 120z) terminate in the manipulator (102) and the end effector (110).

6. The surgical instrument (100) of claim 1, further comprising one additional cable dedicated to operatively coupling the manipulator (102) and the end effector (110).

7. The surgical instrument (100) of claim 1, wherein the first central feature and second central feature each define perpendicular slots, the proximal joint (104) and distal joint (108) first bases and second bases each include projections, and the slots each receive a projection, wherein the projections each define an axis about which the joints (104, 108) can rotate.

8. The surgical instrument (100) of claim 1, further comprising rods (260w, 260x, 260y, 260z) disposed in the hollow elongated member (106) that are coupled to cables (120w, 120x, 120y, 120z) at each end.

9. The surgical instrument (100) of claim 1, wherein the end effector (110) comprises two movable jaws (122, 124) that operate simultaneously.

## Patentansprüche

1. Chirurgisches Instrument (100) zur Verwendung durch einen Operateur, umfassend:
einen Manipulator (102), der zum Aufnehmen zumindest eines Teils einer Hand des Operateurs angepasst ist;
ein proximales Gelenk (104) mit einer ersten Basis und einer zweiten Basis und ein erstes zentrales Merkmal enthaltend, das für eine Gelenkverbindung um zwei rechtwinkelige Achsen sorgt, wobei die erste Basis des proximalen Gelenks (104) am Manipulator (102) montiert ist;
ein hohles längliches Element (106) mit einem ersten Ende, einem zweiten Ende und einer Längsachse, wobei das erste Ende des länglichen Elements (106) an der zweiten Basis des proximalen Gelenks (104) montiert ist;
ein distales Gelenk (108) mit einer ersten Basis und einer zweiten Basis und ein zweites zentrales Merkmal enthaltend, das für eine Gelenkverbindung um zwei rechtwinkelige Achsen sorgt, wobei die erste Basis des distalen Gelenks (108) am zweiten Ende des länglichen Elements (106) montiert ist;
einen Endeffektor (110), der zumindest eine bewegliche Backe (122, 124) enthält, wobei der Endeffektor (110) an der zweiten Basis des distalen Gelenks (108) montiert ist;
Kabel (120w, 120x, 120y, 120z), die mit dem ersten zentralen Merkmal und dem zweiten zentralen Merkmal in Eingriff sind, um den Manipulator (102), das proximale Gelenk (104) und das distale Gelenk (108) betriebsbereit zu koppeln;
wobei das erste zentrale Element und das zweite zentrale Element in ihren Oberflächen Führungen definieren, um die Kabel (120w, 120x, 120y, 120z) aufzunehmen;
**dadurch gekennzeichnet, dass** die Kabel (120w, 120x, 120y, 120z) den Führungen in einem Pfad teilweise um sowohl das erste zentrale Element wie auch das zweite zentrale Element folgen.

2. Chirurgisches Instrument (100) nach Anspruch 1, wobei das erste zentrale Element und das zweite zentrale Element im Wesentlichen kugelförmig sind.

3. Chirurgisches Instrument (100) nach Anspruch 1, wobei die Kabel (120w, 120x, 120y, 120z) zeitgleich den Manipulator (102) und den Endeffektor (110) betriebsbereit koppeln.

4. Chirurgisches Instrument (100) nach Anspruch 2, wobei die Kabel (120w, 120x, 120y, 120z) vier Kabel umfassen.

5. Chirurgisches Instrument (100) nach Anspruch 4, wobei die Kabel (120w, 120x, 120y, 120z) im Manipulator (102) und Endeffektor (110) enden.

6. Chirurgisches Instrument (100) nach Anspruch 1, des Weiteren umfassend ein zusätzliches Kabel, das zum betriebsbereiten Koppeln des Manipulators (102) und Endeffektors (110) bestimmt ist.

7. Chirurgisches Instrument (100) nach Anspruch 1, wobei das erste zentrale Element und das zweite zentrale Element jeweils senkrechte Schlitze definieren, die ersten Basen und zweiten Basis des proximalen Gelenks (104) und des distalen Gelenks (108) jeweils Vorsprünge enthalten und die Schlitze jeweils einen Vorsprung aufnehmen, wobei die Vorsprünge jeweils eine Achse definieren, um die die Gelenke (104, 108) rotieren können.

8. Chirurgisches Instrument (100) nach Anspruch 1, des Weiteren umfassend Stangen (260w, 260x, 260y, 260z), die in dem hohlen länglichen Element (106) angeordnet sind, die an jedem Ende an Kabel (120w, 120x, 120y, 120z) gekoppelt sind.

9. Chirurgisches Instrument (100) nach Anspruch 1, wobei der Endeffektor (110) zwei bewegliche Backen (122, 124) umfasst, die gleichzeitig arbeiten.

## Revendications

1. Un outil chirurgical (100) destiné à être utilisé par un opérateur et comprenant :
un manipulateur (102) apte à recevoir au moins une partie de la main de l'opérateur ;
un joint proximal (104) ayant une première base et une seconde base et incluant un premier élément central qui permet une articulation autour de deux axes perpendiculaires, la première base du joint proximal (104) étant montée sur le manipulateur (102) ;
un élément allongé creux (106) ayant une première extrémité, une seconde extrémité et un axe longitudinal, la première extrémité de l'élément allongé (106) étant montée sur la seconde base du joint proximal (104) ;
un joint distal (108) ayant une première base et une seconde base et incluant un second élément central qui permet une articulation autour de deux axes perpendiculaires, la première base du joint distal (108) étant montée à la seconde extrémité de l'élément allongé (106) ;
un actionneur d'extrémité (110) incluant au moins une mâchoire mobile (122, 124), l'actionneur d'extrémité (110) étant monté sur la seconde base du joint distal (108) ;
des câbles (120w, 120x, 120y, 120z) qui s'engagent dans le premier élément central et le second élément central pour coupler opératoirement le manipulateur (102), le joint proximal (104) et le joint distal (108) ;
les premier élément central et second élément central définissant des guides dans leurs surfaces pour recevoir les câbles (120w, 120x, 120y, 120z) ;
**caractérisé en ce que** les câbles (120w, 120x, 120y, 120z) suivent les guides dans un passage partiellement autour de chacun des premier élément central et second élément central.

2. Outil chirurgical (100) selon la revendication 1, dans lequel le premier élément central et le second élément central ont sensiblement une forme de bille.

3. Outil chirurgical (100) selon la revendication 1, dans lequel les câbles (120w, 120x, 120y, 120z) couplent concurremment et opératoirement le manipulateur (102) et l'actionneur d'extrémité (110).

4. Outil chirurgical (100) selon la revendication 2, dans lequel les câbles (120w, 120x, 120y, 120z) comprennent quatre câbles.

5. Outil chirurgical (100) selon la revendication 4, dans lequel les câbles (120w, 120x, 120y, 120z) se terminent dans le manipulateur (102) et l'actionneur d'extrémité (110).

6. Outil chirurgical (100) selon la revendication 1, comprenant en outre un câble supplémentaire consacré au couplage opératoire du manipulateur (102) et de l'actionneur d'extrémité (110).

7. Outil chirurgical (100) selon la revendication 1, dans lequel le premier élément central et le second élément central définissent chacun des fentes perpendiculaires, les première base et seconde base du joint proximal (104) et du joint distal (108) comprenant chacune des saillies et les fentes recevant chacune une saillie, les saillies définissant chacune un axe autour duquel les joints (104, 108) peuvent tourner.

8. Outil chirurgical (100) selon la revendication 1, comprenant en outre des barres (260w, 260x, 260y, 260z) disposées dans l'élément allongé creux (106) et qui sont couplées aux câbles (120w, 120x, 120y, 120z) à chaque extrémité.

9. Outil chirurgical (100) selon la revendication 1, dans lequel l'actionneur d'extrémité (110) comprend deux mâchoires mobiles (122, 124) qui fonctionnent simultanément.
